Europäisches Patentamt

European Patent Office

Office européen des brevets

(11) Publication number: **0 061 917**
A2

(12) **EUROPEAN PATENT APPLICATION**

(21) Application number: **82301621.7**

(22) Date of filing: **26.03.82**

(51) Int. Cl.³: **D 01 F 4/00**
**A 61 K 37/02, A 61 L 15/00**

(30) Priority: **27.03.81 JP 46022/81**

(43) Date of publication of application:
**06.10.82 Bulletin 82/40**

(84) Designated Contracting States:
**CH DE FR GB IT LI NL SE**

(71) Applicant: **KUREHA KAGAKU KOGYO KABUSHIKI KAISHA**
**9-11 Horidome-cho 1-chome Nihonbashi Chuo-ku Tokyo(JP)**

(72) Inventor: **Kato, Tadaaki**
**K-407, 3-27 Nakadai**
**Itabashi-ku Tokyo(JP)**

(74) Representative: **Myerscough, Philip Boyd et al,**
**J.A.Kemp & Co. 14, South Square Gray's Inn**
**London, WC1R 5EU(GB)**

(54) Collagen fibers for use in medical treatment.

(57) Problems with the quality of collagen fibers prepared by conventional means for use in medicine are overcome by collagen fibers having 312 to 340 glycine residues, 119 to 138 proline residues, 94 to 100 hydroxyproline residues and 2.6 to 5.5 tyrosine residues per 1000 amino acid residues, denaturation temperature of 31 to 40°C, and a S-constant of 1.12 to 1.62. Such fibers, which can be used as a blood-coagulant, are prepared by dispersing a purified collagen, which shows no absorption in the ultraviolet region of the spectrum of wavelength 250-290 nm and which contains less than 0.5% by weight of lipids, in an aqueous acid solution of pH 2 to 4 and irradiating this dispersion at a temperature of not more than 30°C with ultrasonic waves to provide more than 452 kJ of energy per litre of dispersion.

## DESCRIPTION

TITLE: COLLAGEN FIBERS FOR USE IN MEDICAL TREATMENT

The present invention relates to collagen fibers and to a process for their preparation.

Collagen is a protein in corium, cartilage, bone, tendon, etc, and exists in animals in a fibrilar or fiber-like state consisting of bound molecules of collagen about 2800 Å in molecular length and about 15 Å in diameter which is called tropocollagen. Since cross-linking between collagen molecules in an animal body generally increases with the aging of the animal, the major part of collagen is "insoluble collagen" in an old animal. Herein, "insoluble collagen" means collagen which is insoluble in an aqueous solution of an acid or a salt.

In theory the collagen fibers obtained by mechanical fibrating or by chemical treatment of insoluble collagen are capable of being absorbed into a living body (bio-absorbability) and of coagulating blood as well as exhibiting a low antigenicity. It has therefore been expected that such fibers would be used in medicine as an absorbable blood-coagulant and as a material for treating wounds.

The known method of mechanically fibrating collagen entails mechanically and severely crushing insoluble collagen obtained from ox hide and the like. However, even by such mechanical means, the cross-linkages between the molecules of collagen are hardly broken. Consequently, in practice the collagen fibers obtained by mechanical fibrating do not show excellent bio-absorbability and compatibility to the living body.

In addition, the quality of the collagen fibers obtained by mechanical fibrating depends largely on the kind, the sex and the age of the animal from which collagen is derived. As a result, collagen fibers having uniform quality for use in medical treatment can not be always provided. For instance, the isoelectric point of insoluble collagen, the raw material for collagen fibers, is a broad range of from pH 5 to pH 8. The isoelectric point of insoluble collagen is one of the physical constants which indicate the degree of cross-linking between the molecules of collagen, and mainly depends on the kind and the age of the animal. Accordingly, such a variation in the isoelectric point indicates the difficulty in producing insoluble collagen of uniform quality for use in medical treatments.

As a method for solving the problem, it has been proposed to prepare "soluble collagen" fibers by selectively digesting and cutting the cross-linking bonds with a protease without mechanically breaking the molecules of collagen (so-called the method for solubilizing by an enzyme). Herein, "soluble collagen" is collagen which is soluble in an aqueous solution of an acid.

The method for solubilizing by an enzyme gives collagen fibers having improved bio-absorbability and compatability and uniform quality. However, the collagen fibers obtained by the method for solubilizing by an enzyme are not always satisfactory because of their poor blood-coagulating property and high cost due to the use of an expensive and unstable protease and to the inability to re-use the protease. In addition, it is almost impossible to completely remove the protease intermixed into the soluble collagen fibers or to lose the activity of the enzyme without thermally denaturing the solubilized collagen fibers.

Accordingly, it is questionable from the safety aspect to apply soluble collagen fibers obtained by the method for solubilization by an enzyme to living bodies for medical purposes. Further, it is difficult to control the action of the enzyme during digestion and the cutting of the cross-linkages of the raw collagen. Attempts to obtain homogeneity in the product inevitably lead to the production of molecular collagen. The time between the addition of collagen into a blood specimen and the beginning of coagulation of the blood is related to the degree of cross-linking between the molecules of collagen. This period is allegedly very long when molecular collagen is added to blood.

It has now been found that the problems encountered with solubilized collagen fibers obtained by conventional methods are not exhibited by collagen fibers which have 312 to 340 glycine residues, 119 to 138 proline residues, 94 to 100 hydroxyproline residues and 2.6 to 5.5 tyrosine residues per 1000 amino acid residues thereof; a denaturation temperature of 31 to 40°C; and a S-constant of 1.12 to 1.62. These collagen fibers are suitable for use in medical treatment.

The present invention also provides a process for preparing such collagen fibers suitable for use in medical treatment, which process comprises

(i) dispersing purified collagen in an aqueous acid solution of pH 2 to 4, said purified collagen not showing any absorption of ultraviolet rays at wave lengths of 250 to 290 nm and containing less than 0.5% by weight of lipids, and

(ii) irradiating the thus prepared aqueous dispersion with supersonic waves to provide more than 452 kJ (108 kcal) of energy per litre of said aqueous dispersion, the temperature of the aqueous dispersion being not more than 30°C.

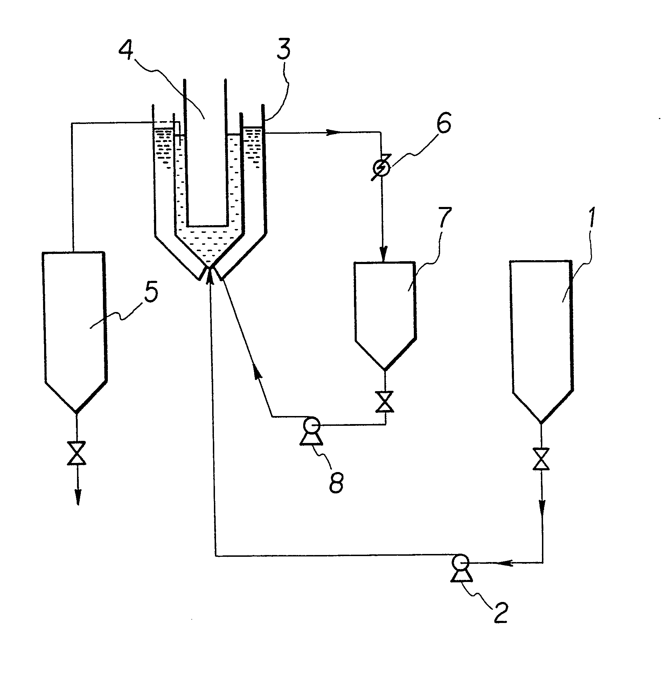

In the description which follows, reference will be made to the accompanying drawing which is a schematic illustration of apparatus for supersonic irradiation of an aqueous acidic dispersion of collagen.

The present inventors have found that the soluble collagen fibers obtained by dispersing the purified collagen in an aqueous acid solution of pH of 2 to 4 and irradiating the aqueous dispersion with a ultrasonic waves to provide more than 108 kcal of energy per litre can be safely used in medical treatment. The fibers exhibit excellent bio-absorbability and velocity of blood-coagulation (the reciprocal of the time between the addition of collagen to a blood specimen and the beginning of blood coagulation). They can be in the form of a powder, freeze-dried porous bodies or a film, and can be used as a blood coagulant.

The reason for the remarkable differences in the properties of the collagen fibers according to the present invention and the properties of solubilized collagen obtained by enzyme-treatment has not yet been elucidated theoretically. However, the difference is presumed to be caused by the difference in homogeneity of the two types of collagen fiber and the difference in the methods for cutting the cross-linking bonds at the terminal parts of molecules of collagen. The difference in the amount of tyrosine residues in the two collagen fibers seems to support the presumption that cutting of cross-linkages by enzyme-treatment occurs as a result of digestion on the telopeptide at the terminal part of collagen molecules and that, on the other hand, cutting according to the process of the present invention occurs physically.

Further, the process according to the present invention is physical in nature. Since enzymes, caustic soda and sodium sulfate are not used in this treatment, there is no fear of their residues being present in the final product. Accordingly, the safety of the product of the present invention as material for medical use can be readily achieved without carrying out procedures for purification to remove such oxygenous impurities. In addition, the product according to the present invention is excellent in reproducibility and easily standardizable.

As the raw material for the soluble collagen fibers according to the present invention, non-denatured connective tissues of warm-blooded animals such as cattle, swine and sheep, for instance, hides, tendones, intestines and bones can be used. Raw hides, refrigerated hides and salted or dried hides are preferred because of their high collagen content.

"Purified collagen" herein is collagen showing no absorption in the ultraviolet region of the spectrum of wave length 250 to 290 nm, and containing less than 0.5% by weight of lipids. The purified collagen is suitably obtained by removing impurities such as proteins other than collagen (such as albumin and globulin), and lipids from crude collagen obtained by subjecting the raw material to conventional pre-treatments such as depilation and decalcification. The absorption of ultraviolet rays in the wave length region of 250 to 290 nm is caused by amino acid residues which are hardly contained in collagen itself. Accordingly, the absorption of ultraviolet rays at from 250 to 290 nm is used as an index of the degree of removal of proteins other than collagen (such as albumin and globulin).

The purified collagen can be prepared from crude collagen by known extraction methods. By repeated immersion of crude collagen in aqueous saline solution, the removal of proteins other than collagen is effected. Lipids can be removed by extraction with a mixed solvent, for instance acetone-ethanol or acetone-ethanol-water. The component of crude collagen which is soluble in an aqueous neutral salt solution may be removed by extracting with a phosphoric acid buffer containing disodium hydrogen phosphate and potassium dihydrogen phosphate. The component of crude collagen which is soluble in acid may be removed by extracting with a citric acid buffer containing citric acid and potassium dihydrogen citrate or a phosphoric acid buffer containing citric acid and disodium hydrogen phosphate. The removal of the soluble components of collagen is carried out until the protein in the extract becomes scarcely detectable by the method of copper-Folin. In addition, pH and concentration of the buffer solutions or the composition of the mixed solvent may be suitably changed according to the components to be removed.

The conditions under which extraction is effected can vary. However, extraction is usually carried out at a temperature lower than 5°C for one to several days. In addition, the order of extraction is not particularly specified, and generally the extraction may be carried out in the following order to prepare purified collagen from raw collagen.

Crude collagen

immersion into an aqueous
10% saline solution for
24 hours

Saline solution (discard) ← | → Collagen

washing with water

Collagen

immersion into an
aqueous 10% saline
solution for 24 hours

Saline solution (discard) ← | → Collagen

washing with water

Collagen

immersion into an
aqueous 10% solution
for 24 hours

Saline solution (discard) ← | → Collagen

washing with water

Collagen

↓ (continued to next page)

0061917

(1) immersion into a mixture of 1:1:2 of acetone, ethanol and water

(2) dehydration and extract with a mixture of 1:1 of acetone and ethanol for 12 hours, 4 times

lipid        Collagen

washing with water
dehydration

Collagen

(1) extract with 1/15M phosphate buffer (pH 8) for 24 hours

Neutral salt-soluble collagen

(2) extract with 0.15M citrate buffer (pH 3.5) for 24 hours, 2 times

Acid-soluble collagen

Collagen

washing with water
substituting water with ethanol
drying under vacuum at lower than 20°C

Purified collagen

The collagen after passing through the purifying steps becomes homogenous fibrous material consisting of insoluble collagen.

In step (i) of the process of the present invention, the pH of the aqueous acid solution used to prepare the aqueous dispersion of collagen is in the range of from 2 to 4. A pH lower than 2 is unfavourable because the swollen and dispersed collagen fibers in the solution separate out from the dispersion. At a pH in the range of from 4 to 10, owing to insufficient swelling of collagen fibers, a homogeneous dispersion is not obtained and collagen precipitates at the bottom of the vessel on standing. On subjecting such a dispersion to ultrasonic wave treatment, collagen fibers begin to coagulate on the surface of the ultrasonic wave oscillator and, finally, all the collagen fibers adhere to the surface as a coagulated lump. In other words, ultrasonic wave treatment at a pH of from 4 to 10 does not finely disperse the collagen fibers, and the appearance and the quality of the thus treated collagen are the same as those of the starting material.

At a pH higher than 10, denaturation of the collagen fibers occurs.

The content of collagen in the aqueous acid dispersion is preferably 0.2 to 10% by weight, more preferably 0.2 to 5% by weight. Where the dispersion contains more than 10% by weight of collagen, the viscosity of the dispersion is too high for it to be handled. On the other hand, where the dispersion contains less than 0.2% by weight of collagen, there is a problem in economic efficiency.

The acid used to make the aqueous acidic solution includes an inorganic acid such as hydrochloric acid, phosphoric acid and sulfuric acid, an organic acid such as acetic acid, butyric acid, citric acid, lactic acid, succinic acid and tartaric acid, and a mixture thereof.

In step (ii) of the process, the amount of energy supplied to the aqueous acidic dispersion by the irradiation of ultrasonic waves is more than 452 kJ (108 kcal) per litre of the aqueous dispersion, preferably from 452 to 10467 kJ ($1.08 \times 10^2$ to $2.5 \times 10^3$ kcal) per litre of the aqueous dispersion. No particular frequency of ultrasonic waves need necessarily be used provided the frequency is in the ultrasonic (supersonic) range. However, the frequency is preferably 17.5 to 24.5 kHz. The amount of energy provided by irradiation with ultrasonic waves depends on the S-constant of the collagen. Namely, the purified raw material, collagen, in the dispersion is fragmented or solubilized as irradiation progresses and ultimately will attain the molecular state. The change of state of the collagen during irradiation can be shown by the determination of the sedimentation velocity of the collagen in the dispersion. The S-constant of collagen fibers in the determination of the sedimentation velocity of collagen fibers is the value concerning the state of the basic units of collagen and a factor concerned with the various physical properties of collagen. The S-constant of the collagen fibers according to the present invention is from 1.12 to 1.62, as measured by apparatus, a MOM 3170/b type tester (manufactured by MOM company, Hungary), at 20°C and at a concentration of collagen of 2 mg/litre.

The irradiation with ultrasonic waves is carried out at a temperature of the aqueous acidic dispersion at which the denaturation of collagen is not caused, namely of not more than 30°C, preferably not more than 20°C

On carrying out irradiation under these conditions, the aqueous dispersion which is at first extremely dense, viscous and opaque becomes less viscous and more translucent as solubilization of collagen by the irradiation proceeds, and finally becomes transparent.

Collagen fibers for use in medical treatment can be easily obtained from the thus irradiated aqueous dispersion by a series of known recovery steps such as neutralization, dialysis, freezing and drying.

The physical properties of the collagen fibers according to the present invention for use in medical treatment are as follows:

(1) amino acid composition of 312 to 340 glycine residues, 119 to 138 proline residues, 94 to 100 hydroxyproline residues and 2.6 to 5.5 tyrosine residues per 1000 total amino acid residues.

(2) temperature of denaturation of 31 to 40°C, and

(3) S-constant of 1.12 to 1.62.

These specific values are close to those of tropocollagen.

By irradiating the purified collagen raw material with ulatrasonic waves, cutting of the cross linkages at the terminal part of the molecules of collagen occurs to produce the collagen fibers excellent in specificities suitable for use in medical treatment. The collagen fibers obtained by the irradiation treatment show a striped pattern with a periodicity of 700 Å specific to collagen under an electron microscope. In addition, the fact that such collagen fibers have not been converted to gelatin is confirmed by the temperature of denaturation from the thus obtained collagen fibers to gelatin. Namely, as has been shown, denaturation of the collagen fibers subjected to the irradiation occurs at a temperature in a range of 31 to 40°C, and the denaturation temperature of the unfibrated corium is 56°C. In addition, the denaturation temperature of the material obtained by mechanically fibrating the unfibrated corium is 46°C, and the soluble collagen obtained by the digestion with enzyme is 31°C.

The temperature of denaturation of collagen can be determined by a scanning-type differential calorimeter (Model DSC-l B, manufactured by Perkin-Elmer Company, USA) as follows:

After introducing 3 mg of air-dried collagen as a specimen into a pressure-resistant and closely sealable cell for DSC-determination and adding 10 microlitres of water, the cell is sealed. After leaving the cell for 2 days at room temperature to enable the specimen to swell, determination is begun by raising the temperature at a rate of 2°C/min. The apex of the DSC-endothermal peak is taken as the "moist-denaturation temperature" of collagen (hereinafter abbreviated to as the denaturation temperature of collagen). In this case, as a control, the same type of cell but only containing 10 microlitres of water is used. The two conditions, i.e. the amount of water introduced into the cell, and the rate of temperature rise have been adopted respectively for the following reasons:

(1) the addition of more than 10 microlitres of water did not affect the denaturation temperature as far as the rate of temperature rise is not changed, and

(2) the dependency of the enaturation temperature on the rate of temperature rise disappears where the rate was lower than 2°C/min.

The aqueous dispersion of irradiated collagen fibers for use in medical treatment can be further processed as follows:

(1) After subjecting the dispersion to the irradiation, the dispersion is treated by neutralization or dialysis to recover the collagen fibers, and the thus recovered collagen fibers are dried, and the lumps thus formed are disintegrated into a powder.

(2) The dispersion subjected to the irradiation is directly freeze-dried to form porous bodies. The thus prepared dried bodies may be disintegrated into a powdery product, or

(3) The dispersion subjected to the irradiation is further subjected to electro-deposition or casting to form a film, which is dried to become a film-like product.

The following Example illustrates the present invention.

EXAMPLE

Fifty grams (dried weight of 12.5 g) of purified raw material for collagen (steer hide, native to North America) prepared by pre-treatment of crude collagen were cut into cubes of about 5 mm in edge length, and immersed in 500 ml of aqueous hydrogen chloride solution of pH 2.5 for one night to cause the collagen to swell. The acid-swollen fragments of collagen were dispersed by a juice mixer into 2.5 litres of aqueous hydrogen chloride solution of pH 3. Then the pH of the dispersion was adjusted to pH 3 with the addition of aqueous hydrogen chloride solution to obtain an aqueous dispersion of 0.5% by weight of collagen.

The thus obtained aqueous dispersion was subjected to the irradiation with ultrasonic waves in the apparatus shown in the accompanying drawing, in which 1 is a feed tank for the dispersion; 2 is a feed pump for the dispersion; 3 is a reactor; 4 is an oscillator of the supersonic waves; 5 is a storage vessel for the treated dispersion; 6 is a cooler for a refrigerant; 7 is a refrigerant tank and 8 is a pump for circulating the refrigerant. This apparatus is provided with an ultrasonic wave generator of 600 W output and 19.5 kHz frequency. The aqueous dispersion is supplied at the apex of the nozzle of the generator, cooling the vessel and keeping the temperature of the aqueous dispersion at 15°C under the respective sets of the conditions shown in Table 1.

The thus obtained acidic dispersions of collagen were freeze-dried to form porous bodies of collagen. Tests on the bio-absorbability of the thus obtained porous bodies of collagen and of comparative collagens (untreated collagen and collagen solubilized by enzyme-treatment) were carried out as follows. The collagen solubilized by enzyme treatment was obtained by first treating the collagen raw material with an aqueous mixed solution of sodium hydroxide and sodium sulfate and then solubilizing the thus treated raw material at pH 9 with Pronase, a protein-hydrolysing enzyme prepared by Kaken Chem. Company, Japan.

Bio-absorbability test

Two of the porous bodies of collagen fibers, each weighing about 5 mg, were administered into the abdominal cavity of male ICR mice 6 to 7 weeks after birth. On the 4th day and on the 7th day after administration, mice were sacrificed. The state of the pieces of collagen fibers that had been administered was observed and recorded.

The time taken for the beginning of blood coagulation of the thus obtained porous bodies of collagen and the control specimens as above was determined by adding each specimen into platelet-rich human plasma and observing coagulation of the blood by a Platelet-aggregation-profiler (Model-PAP-3, manufactured by Bio-Data Company).

The results of the two series of tests are shwon in Table 1. As will be seen in Table 1, the collagen fibers according to the present invention exhibit excellent blood-coagulating property and bio-absorbability as compared to the conventional collagen preparations. Accordingly, the collagen fibers according to the present invention can be used as a hemostatic.

## Table 1

| Classi-fication | No. of speci-men | Energy of supersonic irradiation (kcal/litre) | S-constant | Temperature of denatu-ration (°C) | Main amino acid composition | | | | Time period for blood-coagulation (sec) | Bio-absorbability |
|---|---|---|---|---|---|---|---|---|---|---|
| | | | | | gly-cine | pro-line | hydro-xyroline | tyro-sine | | |
| Present invention | 1 | 175 | 1.55 | 35 | 335 | 122 | 96 | 5.0 | 117.6 | partly remained on 4th day and all absorbed on 7th day |
| | 2 | 1290 | 1.17 | 34 | 338 | 120 | 97 | 4.9 | 120.0 | all absorbed on 4th day |
| | 3 | 2700 | 1.12 | 34 | 340 | 121 | 97 | 5.0 | 130.2 | all absorbed on 4th day |
| Compara-tive Ex. | 4 | 30 | 1.71 | 42 | 337 | 123 | 97 | 4.8 | 86.3 | all remained on 4th day and partly remained on 7th day |
| | 5(1) | - | 1.82 | 56 | 340 | 122 | 96 | 4.9 | 32.4 | remained on 7th day |
| | 6(2) | - | 1.10 | 31 | 337 | 122 | 96 | 0.5 | 146.4 | absorbed on 4th day |

Note: (1) non-treated collagen (the raw material of the present invention) and

(2) collagen solubilized by enzyme-treatment.

CLAIMS

1.  Collagen fibers suitable for use in medical treatment, said fibers having 312 to 340 glycine residues, 119 to 138 proline residues, 94 to 100 hydroxyproline residues and 2.6 to 5.5 tyrosine residues per 1000 amino acid residues; a denaturation temperature of 31 to 40°C; and a S-constant of 1.12 to 1.62.

2.  Collagen fibers according to claim 1 for use as a blood-coagulant.

3.  A powder, freeze-dried porous bodies or a film derived from collagen fibers as claimed in claim 1.

4.  A process for preparing collagen fibers suitable for use in medical treatment, which process comprises
(i) dispersing purified collagen in an aqueous acid solution of pH 2 to 4, said purified collagen showing no absorption in the ultraviolet region of the spectrum of wave length of from 250 to 290 nm and containing less than 0.5% by weight of lipids, and
(ii) irradiating the thus prepared aqueous dispersion with ultrasonic waves to provide more than 452 kJ of energy per litre of said aqueous dispersion, the temperature of the aqueous dispersion being not more than 30°C.

5.  A process according to claim 4, wherein the purified collagen is dispersed in such an amount in step (i) that the resulting aqueous dispersion contains from 0.2 to 10% by weight of the purified collagen.